# EUROPEAN PATENT SPECIFICATION

(11) **EP 2 098 173 B1**
(45) Date of publication and mention of the grant of the patent: **19.03.2014**
(21) Application number: 07851053.4
(22) Date of filing: 21.12.2007
(51) Int. Cl.: A61B 17/08, A61L 17/00, A61L 17/12, A61L 17/14, A61B 17/04

(54) **STITCHING REINFORCEMENT MATERIAL FOR AUTOMATIC STITCHING DEVICE**
NAHTVERSTÄRKUNGSMATERIAL FÜR EIN AUTOMATISCHES NÄHGERÄT
MATÉRIAU DE RENFORCEMENT DE COUTURE POUR UN DISPOSITIF DE COUTURE AUTOMATIQUE

(30) Priority: 25.12.2006 JP 2006347967
(43) Date of publication of application: 09.09.2009
(73) Proprietor: Gunze Limited, Ayabe-shi, Kyoto 623-8511 (JP)
(72) Inventor: MATSUDA, Shojiro, Ayabe-shi Kyoto 623-8512 (JP); SAKAMOTO, Yuki, Ayabe-shi Kyoto 623-8512 (JP)
(74) Representative: Albrecht, Thomas
(86) International application number: PCT/JP2007/074701
(87) International publication number: WO 2008/078697

(56) References cited:
- WO-A1-00/56376
- JP-A- 09 024 050
- JP-A- 10 216 217
- JP-A- 10 216 217
- JP-A- 2001 286 477
- US-A- 5 843 096

## Description

### TECHNICAL FIELD

The present invention relates to a suture reinforcement material for an automatic suturing device, which has excellent loadability into an automatic suturing device and excellent cuttability with a cutter.

### BACKGROUND ART

Excision using an automatic suturing device is performed as a method to remove a lesion occurring in living tissues under an endoscope. After introduced in a living body from a perforated hole, an automatic suturing device is manipulated to excise the affected area and to simultaneously suture the excision site. Use of the automatic suturing device is advantageous in that incision of the affected area is not necessary. The automatic suturing device has a suturing section, which clamps the excision site of the living tissues, at a tip end, and the suturing section is provided with a plurality of staple lines for suturing and an excising mechanism of living tissues (for example, cutter).

Upon excision of a fragile organ such as lung, bronchi, liver and gastrointestinal tract with an automatic suturing device, suturing only with a staple may tear the organ. Further, in a case of a lung surgery, air leakage may occur. Therefore, a suture reinforcement material previously loaded into the automatic suturing device is sutured with staples to the excision site of the living tissues.

The suture reinforcement material to be used in the automatic suturing device needs to have various characteristics such as easy loading into the automatic suturing device, no misalignment after being loaded, and good cuttability with a cutter, in addition to the capabilities to prevent tearing of the organs and air leakage.

Patent Document 1 and other documents have disclosed suture reinforcement materials formed of a nonwoven fabric, a woven fabric or a knit fabric as the suture reinforcement material to be used in an automatic suturing device. Although those suture reinforcement materials formed of a nonwoven fabric and the like can be cut with a cutter, the cutting is problematically difficult. Moreover, improvement has been desired concerning the air leakage. On the other hand, Patent Document 2 and other documents have disclosed suture reinforcement materials which are formed of a foamed film. The suture reinforcement materials formed of a foamed film have superior cuttability with a cutter to the suture reinforcement material formed of a nonwoven fabric, and thus those materials can be easily cut out and removed. Furthermore, the suture reinforcement materials are excellent in preventing air leakage. However, since the suture reinforcement material formed of a foamed film has poor slidability, loading of the material into the automatic suturing device is problematically difficult.
Patent Document 1: Japanese Patent No. 3795100 (JP-B 3795100)
Patent Document 2: Japanese Patent No. 3237750 (JP-B 3237750)

### DISCLOSURE OF THE INVENTION

### PROBLEMS TO BE SOLVED BY THE INVENTION

In view of the above-mentioned state of the art, the present invention aims to provide a suture reinforcement material for an automatic suturing device, which has excellent loadability into an automatic suturing device and excellent cuttability with a cutter.

### MEANS FOR SOLVING THE PROBLEMS

The present invention is a suture reinforcement material for an automatic suturing device, which comprises a tubular body comprising an inner layer and an outer layer, the inner layer comprising a nonwoven fabric, the outer layer comprising a foamed polymer, wherein the nonwoven fabric of the inner layer and the foamed polymer of the outer layer are integrated together.

The following description will discuss the present invention in more detail.

The suture reinforcement material for an automatic suturing device according to the present invention (hereinafter, also simply referred to as suture reinforcement material) comprises a tubular body including an inner layer formed of a nonwoven fabric and an outer layer formed of a foamed polymer. Since the inner layer is formed of a nonwoven fabric which has excellent slidability, the suture reinforcement material of the present invention can be extremely easily loaded into the automatic suturing device. The nonwoven fabric has an effect to maintain the shape of the suture reinforcement material of the present invention as well. The suture reinforcement material further has excellent cuttability with a cutter due to the presence of the outer layer formed of a foamed polymer integrated with the inner layer formed of a nonwoven fabric. Moreover, the presence of the foamed polymer as the outer layer provides the suture reinforcement material of the present invention with high resiliency, mild contact to the affected area, and excellent staple penetrability.

The phrase, the nonwoven fabric and the foamed polymer are integrated together described herein means that the foamed polymer infiltrates into at least a part of the surface fabric of the nonwoven fabric so that the nonwoven fabric layer and the foamed polymer layer are at least partly attached together to form an inseparable state.

The nonwoven fabric is not particularly limited, and examples thereof include nonwoven fabrics having a weight per unit area of 10 g/m² in the preferable lower limit and 35 g/m² in the preferable upper limit. A nonwoven fabric having a weight per unit area of less than 10 g/m² may not exert the slidability-providing effect. A nonwoven fabric having a weight per square-meter of more than 35 g/m² may produce a suture reinforcement material having inferior flexibility. The more preferable lower limit is 15 g/m² and the more preferable upper limit is 30 g/m².

The thickness of the nonwoven fabric is preferably 0.01 mm in the lower limit and 0.3 mm in the upper limit, though not particularly limited to this range. The thickness of less than 0.01 mm may fail to practically produce a nonwoven fabric, and the thickness of more than 0.3 mm may produce a suture reinforcement material having an excessive total thickness. The more preferable lower limit is 0.05 mm and the more preferable upper limit is 0.2 mm.

The porosity of the foamed polymer is preferably 60% in the lower limit and 95% in the upper limit, though not particularly limited to this range. The porosity of lower than 60% may produce a suture reinforcement material having inferior flexibility. The porosity of higher than 95% may produce a suture reinforcement material having inferior strength or an insufficient air leakage-prevention effect. The more preferable lower limit is 70% and more preferable upper limit is 90%.

The thickness of the foamed polymer is preferably 0.01 mm in the lower limit and 0.5 mm in the upper limit, though not particularly limited to this range. The thickness of less than 0.01 mm may fail to practically form a foamed polymer. The thickness of more than 0.5 mm may produce a suture reinforcement material having an excessive total thickness. The more preferable lower limit is 0.05 mm and the more preferable upper limit is 0.3 mm.

The nonwoven fabric and the foamed polymer are each preferably formed of a bioabsorbable material. Since the nonwoven fabric and the foamed polymer made of bioabsorbable materials are decomposed and absorbed within a certain period of time from the operation, they need not to be removed out by performing a follow-up operation. Accordingly, a burden to the patient can be significantly reduced.

Examples of the bioabsorbable materials constituting the nonwoven fabric and the foamed polymer include polyglycolic acid, polylactic acid (D, L, DL), polycaprolactone, a copolymer of glycolic acid and lactic acid (D, L, DL), a copolymer of glycolic acid and ε-caprolactone, a copolymer of lactic acid (D, L, DL) and ε-caprolactone, poly (p-dioxanone), and other materials. Those materials maybe used solely or in combination of two or more kinds thereof. Polyglycolic acid and a copolymer of glycolic acid and ε-caprolactone are especially preferable as the bioabsorbable materials for the nonwoven fabric because those materials have an excellent flexibility, resilience, staple line-formability, hydrolyzability, and the like. A copolymer of lactic acid (D, L, DL) and ε-caprolactone and a copolymer of glycolic acid and ε-caprolactone are especially preferable as the bioabsorbable materials for the foamed polymer because those materials have an excellent flexibility.

The cross-sectional shape of the tubular body of the suture reinforcement material according to the present invention may be a circular shape or the same shape as a cross-sectional shape of the automatic suturing device so as to achieve better loading. Moreover, the tip portion may be in a tapered shape or may be in the form of a closed bag.

The suture reinforcement material of the present invention is preferably provided with a perforated line so as to facilitate detachment of a suture reinforcing portion to be left in the body from a portion to be removed. Moreover, in the suture reinforcement material of the present invention, preferably end parts of the inner layer formed of the nonwoven fabric are superimposed on one another to configure a tubular shape so as to facilitate detachment of the suture reinforcing portion to be left in the body from the portion to be removed. Furthermore, the suture reinforcement material of the present invention preferably has a tractive string to remove the portion to be removed out of the body.

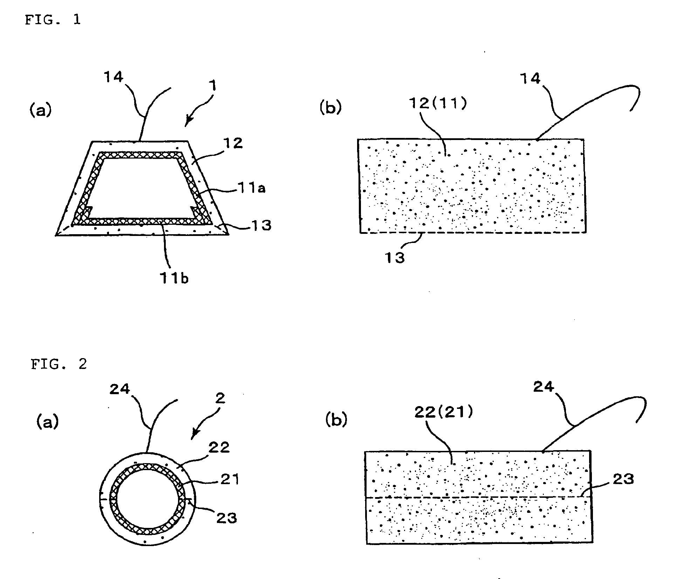

Figs. 1 and 2 illustrate one embodiment of the suture reinforcement material of the present invention.

Fig. 1(a) is a cross-sectional view and Fig. 1(b) is a side view of one embodiment of the suture reinforcement material of the present invention, which is to be loaded on the cartridge side of an automatic suturing device. The suture reinforcement material 1 shown in Fig. 1 has a tubular body including an inner layer 11 formed of a nonwoven fabric and an outer layer 12 formed of a foamed polymer. The shape of the cross section is the same as the shape of the cross section of the automatic suturing device. The outer layer 12 formed of a foamed polymer is provided with a perforated line 13. The inner layer 11 formed of a nonwoven fabric consists of two nonwoven fabric layers 11a and 11b. The end parts of the nonwoven fabric layer 11a are superimposed on the respective end parts of the nonwoven fabric layer 11b, and the nonwoven fabric layer 11a is separable from the nonwoven fabric layer 11b at positions of the perforated lines 13 in the outer layer 12 formed of a foamed polymer. The suture reinforcement material 1 is further provided with a tractive string 14 to be pulled after the operation to facilitate the removal of unnecessary parts from inside the body.

Fig. 2(a) is a cross-sectional view and Fig. 2(b) is a side view of another embodiment of the suture reinforcement material of the present invention, which is to be loaded on the anvil side of the automatic suturing device. A suture reinforcement material 2 shown in Fig. 2 has a tubular body with a circular cross section consisting of an inner layer 21 and an outer layer 22, the inner layer 21 being formed of a nonwoven fabric which has been previously configured in the tubular shape, the outer layer 22 being formed of a foamed polymer. The suture reinforcement material 2 is provided with a perforated line 23 and further a tractive string 24. In this configuration, the end parts of the reinforcement materials may be superimposed in a similar manner as shown in Fig. 1.

A method for producing the suture reinforcement material of the present invention is not particularly limited, and examples of the method are: a method (freeze-drying method) including fixing a previously prepared nonwoven fabric to a suitable substrate such as a rod made of a fluorine resin, immersing the resulting product in a solution of a bioabsorbable material, followed by freeze-drying; a method (elution method) including attaching a mixed solution of a water-soluble material and the bioabsorbable material forming the foamed polymer to a previously prepared nonwoven fabric, and then washing out the water-soluble material with water; and the like. When the freeze-drying method is employed, it is possible to prepare foamed polymers having various pore diameters by controlling freezing temperatures, the concentration of the polymer, and the like. When the elution method is employed, it is possible to control the pore diameter of the foamed polymers by adjusting the particle of the water-soluble material.

Alternatively, the suture reinforcement material of the preset invention may be produced by other methods, such as a method including preparing a rectangular sheet consisting of a laminated body of the nonwoven fabric and the foamed polymer, and sawing the sheet into the form of a bag.

### EFFECT OF THE INVENTION

In accordance with the present invention, it is possible to provide a suture reinforcement material for an automatic suturing device, which has excellent loadability into an automatic suturing device and excellent cuttability with a cutter.

### BEST MODE FOR CARRYING OUT THE INVENTION

Hereinafter, the present invention will be described in more detail by showing examples; however, the present invention is not limited to these examples.

### (Example 1)

A nonwoven fabric made of a polyglycol acid having a weight per unit area of 30 g/m² and a thickness of 0.2 mm was prepared by a melt-blow method.

As illustrated in Fig. 1, two sheets of the nonwoven fabric were wound around a rod made of a fluorine resin and having generally the same cross-sectional shape and the size as the cartridge side of the automatic suturing device. End parts of one sheet of the nonwoven fabric were superimposed on respective end parts of the other nonwoven fabric sheet. Peripheral ends were temporarily fixed with a tape. The resulting product was frozen at -80 °C for one hour or more.

The product was made longer than the length of the area to be provided with staples of a stapler, taking into consideration that the areas temporarily fixed with a tape were to be cut off later.

The nonwoven fabric fixed onto the rod was immersed in a 4 wt% solution of a copolymer of L-lactic acid and ε-caprolactone (molecular ratio 50:50) in dioxane, and slowly taken out. The resulting product was frozen at a temperature of -80°C, freeze-dried at a temperature of -40°C to 40°C for 12 hours, and then subjected to heat treatment at a temperature of 70°C for 12 hours to provide a suture reinforcement material. The thus-obtained suture reinforcement material included the nonwoven fabric on the inner side and the foamed polymer on the outer side, with the nonwoven fabric and the foamed polymer integrated together, and had the same cross sectional shape as that of the cartridge side of the automatic suturing device.

### (Comparative Example 1)

The nonwoven fabric prepared in Example 1 was wound around a rod made of a fluorine resin and having generally the same cross-sectional shape and the size as the cartridge side of the automatic suturing device, and then fixed with a tape to produce a suture reinforcement material consisting only of a nonwoven fabric.

### (Comparative Example 2)

A rod made of a fluorine resin having a cross-sectional shape identical to the cartridge side of the cross-sectional shape of the automatic suturing device was immersed in a 4 wt% solution of a copolymer of L-lactic acid and ε-caprolactone (molecular ratio 50:50) in dioxane, and slowly taken out. The rod was frozen at a temperature of -80°C, freeze-dried at a temperature of -40°C to 40°C for 12 hours, and then subjected to heat treatment at a temperature of 70°C for 12 hours to provide a suture reinforcement material consisting only of a foamed polymer.

The suture reinforcement materials produced in Example 1, Comparative Example 1, and Comparative Example 2 were evaluated on loadability into the automatic suturing device and cuttability with a cutter according to the methods described below.

Table 1 shows the results.

### (1) Loadability into an Automatic Suturing Device

Each of the suture reinforcement materials was manually loaded on the cartridge side of an automatic suturing device. The loadability at the time was evaluated based on the following criteria:
o: Good slidability, leading to easy loading
x: Bad slidability, leading to a longer loading time and damage to the suture reinforcement material

### (2) Cuttability with Cutter

The suture reinforcement material loaded on the cartridge side of the automatic suturing device was cut with a cutter by manipulating the automatic suturing device. The cuttability at the time was evaluated based on the following criteria:
o: Cut easily and linearly
x: Uncut parts remained, Unable to be cut linearly

**[Table 1]**

| | Composition | Evaluation | |
|---|---|---|---|
| | | Loadability | Cuttability |
| Example 1 | Nonwoven fabric (inner layer) /Foam (outer layer) | ○ | ○ |
| Comparative Example 1 | Nonwoven fabric only | ○ | × |
| Comparative Example 2 | Foam only | × | ○ |

### INDUSTRIAL APPLICABILITY

According to the present invention, it is possible to provide a suture reinforcement material for an automatic suturing device, which has excellent loadability into an automatic suturing device and excellent cuttability with a cutter.

### BRIEF DESCRIPTIONS OF THE DRAWINGS

Fig. 1(a) is a cross-sectional view and Fig. 1(b) is a side view of one embodiment of the suture reinforcement material of the present invention to be loaded on the cartridge side of an automatic suturing device.
Fig. 2(a) is a cross-sectional view and Fig. 2(b) is a side view of one embodiment of the suture reinforcement material of the present invention to be loaded on the anvil side of an automatic suturing device.

### EXPLANATION OF SYMBOLS

- 1: Suture reinforcement material
- 11a, 11b: Inner layer formed of a nonwoven fabric
- 12: Outer layer formed of foamed polymer
- 13: Perforated line
- 14: Tractive string
- 2: Suture reinforcement material
- 21: Inner layer formed of nonwoven fabric
- 22: Outer layer formed of foamed polymer
- 23: Perforated line
- 24: Tractive string

## Claims

1. A suture reinforcement material for an automatic suturing device,
which comprises a tubular body comprising an inner layer and an outer layer, the inner layer comprising a nonwoven fabric, the outer layer comprising a foamed polymer, wherein
the nonwoven fabric of the inner layer and the foamed polymer of the outer layer are integrated together, wherein integrated together means that the foamed polymer infiltrates into at least a part of the surface fabric of the nonwoven fabric so that the nonwoven fabric layer and the foamed polymer layer are at least partly attached together to form an inseparable state.

2. The suture reinforcement material for an automatic suturing device according to claim 1,
wherein the nonwoven fabric and the foamed polymer each comprises a bioabsorbable material.

3. The suture reinforcement material for an automatic suturing device according to claim 1,
wherein the bioabsorbable material constituting the nonwoven fabric or the foamed polymer is at least one species selected from the group consisting of polyglycolic acid, polylactic acid (D, L, DL), polycaprolactone, a copolymer of glycolic acid and lactic acid (D, L, DL), a copolymer of glycolic acid and ε-caprolactone, a copolymer of lactic acid (D, L, DL) and ε-caprolactone, and poly(p-dioxanone).

4. The suture reinforcement material for an automatic suturing device according to claim 1, 2, or 3,
wherein a cross-sectional shape of the tubular body is a circular shape or the same shape as a cross-sectional shape of the automatic suturing device.

5. The suture reinforcement material for an automatic suturing device according to claim 1, 2, 3, or 4,
which is provided with a perforated line so as to facilitate detachment of a suture reinforcing portion to be left in the body from a portion to be removed.

6. The suture reinforcement material for an automatic suturing device according to claim 1, 2, 3, 4, or 5,
wherein end parts of the inner layer comprising the nonwoven fabric are superimposed on one another to configure a tubular shape so as to facilitate detachment of a suture reinforcing portion to be left in the body from a portion to be removed.

7. The suture reinforcement material for an automatic suturing device according to claim 1, 2, 3, 4, 5, or 6,
which comprises a tractive string to remove the portion to be removed out of the body.

## Patentansprüche

1. Nahtverstärkungsmaterial für ein automatisches Nähgerät,
das einen schlauchförmigen Körper umfasst, der eine innere Schicht und eine äußere Schicht umfasst, wobei die innere Schicht ein Nonwoven-Gewebe umfasst, die äußere Schicht ein geschäumtes Polymer umfasst, wobei
das Nonwoven-Gewebe der inneren Schicht und das geschäumte Polymer der äußeren Schicht miteinander integriert sind, wobei miteinander integriert bedeutet, dass das geschäumte Polymer in wenigstens einen Teil des Oberflächengewebes des Nonwoven-Gewebes infiltriert, so dass die Nonwoven-Gewebe-Schicht und die geschäumte Polymerschicht wenigstens teilweise unter Bildung eines untrennbaren Zustandes miteinander verbunden sind.

2. Nahtverstärkungsmaterial für ein automatisches Nähgerät gemäß Anspruch 1,
wobei das Nonwoven-Gewebe und das geschäumte Polymer jeweils ein bioabsorbierbares Material umfassen.

3. Nahtverstärkungsmaterial für ein automatisches Nähgerät gemäß Anspruch 1,
wobei das bioabsorbierbare Material, das das Nonwoven-Gewebe oder das geschäumte Polymer bildet, wenigstens eine Spezies, ausgewählt aus der Gruppe, bestehend aus Polyglycolsäure, Polymilchsäure (D, L, DL), Polycaprolacton, einem Copolymer aus Glycolsäure und Milchsäure (D, L, DL), einem Copolymer aus Glycolsäure und ε-Caprolacton, einem Copolymer aus Milchsäure (D, L, DL) und ε-Caprolacton und Poly(p-dioxanon), ist.

4. Nahtverstärkungsmaterial für ein automatisches Nähgerät gemäß Anspruch 1, 2 oder 3,
wobei die Querschnittsform des schlauchförmigen Körpers eine kreisförmige Form oder dieselbe Form wie die Querschnittsform des automatischen Nähgerätes ist.

5. Nahtverstärkungsmaterial für ein automatisches Nähgerät gemäß Anspruch 1, 2, 3 oder 4,
das mit einer perforierten Linie bereitgestellt wird, um so eine Ablösung eines nahtverstärkenden Teils, der in dem Körper zurückbleibt, von einem zu entfernenden Teil zu erleichtern.

6. Nahtverstärkungsmaterial für ein automatisches Nähgerät gemäß Anspruch 1, 2, 3, 4 oder 5,
wobei Endteile der inneren Schicht, die das Nonwoven-Gewebe umfasst, übereinander gelegt sind, um eine schlauchförmige Form zu konfigurieren, um so eine Ablösung eines nahtverstärkenden Teils, der in dem Körper zurückbleiben soll, von einem zu entfernenden Teil zu erleichtern.

7. Nahtverstärkungsmaterial für ein automatisches Nähgerät gemäß Anspruch 1, 2, 3, 4, 5 oder 6,
das einen Zugfaden umfasst, um den Teil zu entfernen, der aus dem Körper entfernt werden soll.

## Revendications

1. Matériau de renforcement de suture pour un dispositif de suture automatique,
qui comprend un corps tubulaire comprenant une couche interne et une couche externe, la couche interne comprenant une étoffe non tissée, et la couche externe comprenant un polymère expansé, dans lequel
l'étoffe non tissée de la couche interne et le polymère expansé de la couche externe sont intégrés ensemble, dans lequel « intégrés ensemble » signifie que le polymère expansé s'infiltre dans au moins une partie de l'étoffe de surface de l'étoffe non tissée de sorte que la couche d'étoffe non tissée et la couche de polymère expansé sont au moins partiellement attachées ensemble afin de former un état inséparable.

2. Matériau de renforcement de suture pour un dispositif de suture automatique selon la revendication 1,
dans lequel l'étoffe non tissée et le polymère expansé comprennent chacun un matériau bioabsorbable.

3. Matériau de renforcement de suture pour un dispositif de suture automatique selon la revendication 1,
dans lequel le matériau bioabsorbable constituant l'étoffe non tissée ou le polymère expansé est au moins une espèce choisie dans le groupe consistant en le poly(acide glycolique), le poly(acide lactique) (D, L, DL), la poly(caprolactone), un copolymère d'acide glycolique et d'acide lactique (D, L, DL), un copolymère d'acide glycolique et d'ε-caprolactone, un copolymère d'acide lactique (D, L, DL) et d'ε-caprolactone, et la poly(p-dioxanone).

4. Matériau de renforcement de suture pour un dispositif de suture automatique selon la revendication 1, 2 ou 3,
dans lequel une forme en coupe du corps tubulaire est une forme circulaire ou la même forme qu'une forme en coupe du dispositif de suture automatique.

5. Matériau de renforcement de suture pour un dispositif de suture automatique selon la revendication 1, 2, 3 ou 4,
qui est doté d'une ligne perforée de façon à faciliter le détachement d'une portion de renforcement de suture devant être laissée dans le corps d'une portion devant être enlevée.

6. Matériau de renforcement de suture pour un dispositif de suture automatique selon la revendication 1, 2, 3, 4 ou 5,
dans lequel les parties d'extrémité de la couche interne comprenant l'étoffe non tissée sont superposées les unes sur les autres afin de configurer une forme tubulaire de façon à faciliter le détachement d'une portion de renforcement de suture devant être laissée dans le corps dune portion devant être enlevée.

7. Matériau de renforcement de suture pour un dispositif de suture automatique selon la revendication 1, 2, 3, 4, 5 ou 6,
qui comprend un cordon de traction afin d'enlever la portion devant être enlevée du corps.
